# EUROPEAN PATENT APPLICATION

(11) **EP 0 068 669 A1**
(43) Date of publication of application: **05.01.1983**
(21) Application number: 82302930.1
(22) Date of filing: 08.06.1982
(51) Int. Cl.: C07C 91/16, C07C 143/75, C07C 125/065, C07C 103/44, C07C 127/19, C07C 101/18, C07D 295/12, C07D 211/62, A61K 31/135, A61K 31/16, A61K 31/18, C07C 103/42, C07C 87/62, C07C 97/10, C07D 295/06, C07D 295/10

(54) **Secondary phenylethanol amines, processes for their preparation and their pharmaceutical application**

(30) Priority: 20.06.1981 GB 8119112
(71) Applicant: BEECHAM GROUP PLC, Brentford, Middlesex TW8 9BD (GB)
(72) Inventor: Duckworth, David Malcolm, Tadworth Surrey (GB); Hindley, Richard Mark, Reigate Surrey (GB)
(74) Representative: Cresswell, Thomas Anthony

(57) **Abstract**

Compounds of formula (I) 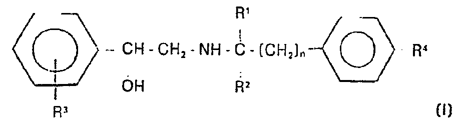 and salts and in vivo hydrolysable acyl derivatives thereof wherein
R' is hydrogen or methyl;
R² is hydrogen or methyl;
R³ is hydrogen, fluarine, chlorine, bromine, trifluoromethyl or C₁₋₆ straight or branched chain alkyl;
R⁴ is nitro, 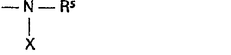
or optionally substitutec 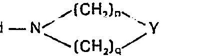
R^{S} is hydrogen or C₁₋₆ straight or branched chain alkyl;
X is R⁶; 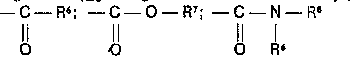 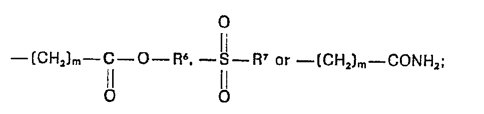
R⁶ is hydrogen or C₁₋₆ straight or branched chain alkyl;
R⁷ is C₁₋₆ straight or branched chain alkyl;
R⁸ is hydrogen or C₁₋₆ straight or branched chain alkyl;
m is an integer from 1 to 6;
p is an integer from 2 to 5;
q is an integer from 0 to 2;
provided that p + q is from 3 to 5;
Y is 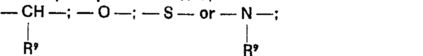
R⁹ is hydrogen, C₁₋₆ straight or branched alkyl or aralkyl consisting of a C₂ or C₁₀ aryl group and a C₁₋₆ straight or branched chain alkylene group;
any substituent on the 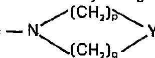 group when present, being a carboxyl group or a salt, ester or amide thereof.
   have anti-obesity, and/or hypoglycaemic and/or antiin- flammation and/or platelet aggregation inhibiting activity.

## Description

The present invention relates to derivatives of ethanolamine which have anti-obesity and/or hypoglycaemic and/or_{'}anti-inflammatory and/or platelet aggregation inhibition activity, to processes for their production and to their use in medicine.

European Patent Application No. 79301197.4 (Beechams) discloses compounds of formula 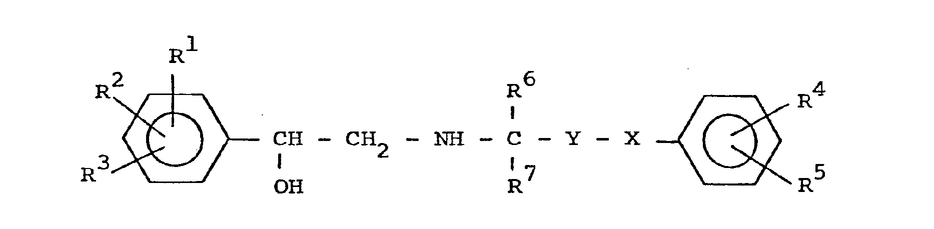
wherein R¹ is hydrogen, fluorine, chlorine, hydroxyl, hydroxymethyl, methyl, methoxy, amino, formamido, acetamido, methylsulphonamido, nitro, benzyloxy, methylsulphonylmethyl, ureido, trifluoromethyl or p-methoxybenzylamino;
R² is hydrogen, fluorine, chlorine or hydroxyl;
_{R}³ is hydrogen, fluorine, chlorine or hydroxyl;
R⁴ is a carboxylic acid group or a salt, ester or amide thereof;
_{R}⁵ is hydrogen, fluorine, chlorine, methyl, methoxy, hydroxyl, or a'carboxylic acid group or a salt, ester or amide thereof;
R⁶ is hydrogen, methyl, ethyl or propyl;
R is hydrogen, methyl, ethyl or propyl;
X is oxygen or a bond; and
Y is C₁₋₆ alkylene or a bond,
which possess anti-obesity and/or hypoglycaemic activity.

It has now been discovered that a class of novel anilinoethanolamine derivatives have anti-obesity and/or hypoglycaemic and/or anti-inflammatory and/or platelet aggregation inhibition activity. This activity is coupled with low cardiac stimulant activity for particular members of the class.

Accordingly, the present invention provides a compound of formula (I): 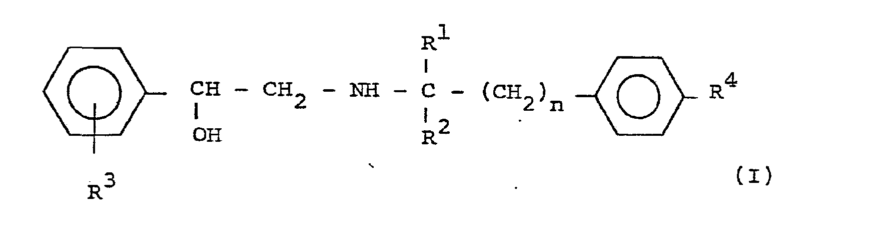 and salts and in vivo hydrolysable acyl derivatives thereof wherein
_{R}¹ is hydrogen or methyl;
R² is hydrogen or methyl;
_{R}³ is hydrogen, fluorine, chlorine, bromine, trifluoromethyl or C₁₋₆ straight or branched chain alkyl;
R⁴ is nitro, 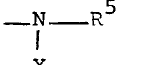 or optionally substituted 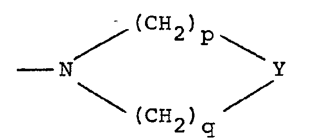
R5 is hydrogen or C₁₋₆ straight or branched chain alkyl;
X is _{R}6_{;} 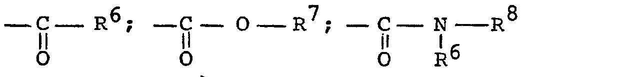 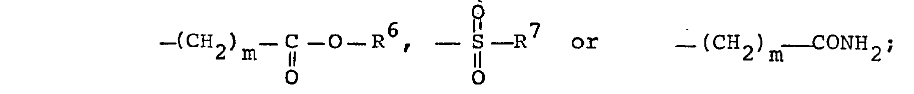
R⁶ is hydrogen or Cₗ-₆ straight or branched chain alkyl;
R⁷ is C₁₋₆ straight or branched chain alkyl;
R⁸ is hydrogen or C₁₋₆ straight or branched chain alkyl;
m is an integer from 1 to 6;
p is an integer from 2 to 5;
q is an integer from 0 to 2;
provided that p + q is from 3 to 5;
Y is 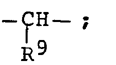 -O-; -S - or 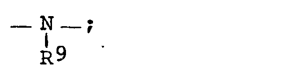
R⁹ is hydrogen, C₁₋₆ straight or branched alkyl or aralkyl consisting of a C₆ or C₁₀ aryl group and a C₁₋₆ straight or branched chain alkylene group;
any substituent on the 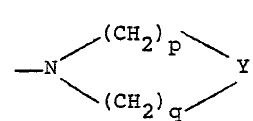 group, when present, being a carboxyl group or a salt, ester or amide thereof.

Pharmaceutically acceptable salts of compounds of formula (I) include acid addition salts formed with a pharmaceutically acceptable acid such as hydrochloric acid, hydrobromic acid, orthophosphoric acid, sulphuric acid, methane sulphonic acid, toluenesulphonic acid, acetic acid, propionic acid, lactic acid, citric acid, fumaric acid, malic acid, succinic acid, salicylic acid or acetylsalicyclic acid; and, when R⁴ includes a carboxyl group, salts of that group such as alkali metal salts, including sodium and potassium salts, alkaline earth metal salts, including calcium and magnesium salts, and ammonium salts.

The salts of compounds of formula (I) need not be pharmaceutically acceptable as they are also useful in the preparation of other compounds of formula (I) and in the separation of stereoisomers of compounds of formula (I) when the salt ion is also optically active.

In vivo hydrolysable acyl derivatives are those which are converted to compounds of formula (I) in the body of a patient to whom the acyl derivative has been administered.

Compounds of formula (I) have at least one asymmetric carbon atom, ie the one bearing the hydroxyl and substituted phenyl groups, and, when R¹ and R² are different, the carbon atom bearing R¹ and R² is also asymmetric. The compounds may therefore exist in at least two and often four stereoisomeric forms. The present invention encompasses all stereoisomers of the compounds of formula (I) whether free from other stereoisomers or admixed with other stereoisomers in any proportion and thus includes, for instance, racemic mixtures of enantiomers.

Preferably, the carbon atom bearing the hydroxyl and substituted phenyl groups has the R configuration.

The most potent compounds of formula (I) are those wherein R¹ and R² are different and both asymmetric carbon atoms are in the R configuration.

The absolute configuration of any compound of formula (I) may be determined by conventional X-ray crystallographic techniques.

It is believed that, in the ¹³C n.m.r. spectrum of compounds of formula (I) wherein one of R and _{R}² is hydrogen and the other is methyl, the diastereoisomer having the greater anti-obesity and hypoglycaemic activity is that for which the signal of the methyl group carbon atom appears at higher field (the lower numerical value when expressed in ppm) in d₆DMSO solution. The paired resonances often appear at approximately 20 ppm (less active) and slightly below 20 ppm (more active) down field from tetramethylsilane. Other paired resonances can occur for the carbon atoms attached directly to the nitrogen atom and the carbon which carries the hydroxyl and phenyl groups. Again the paired resonances of the more active diastereoisomer of the investigated compounds appear at the higher field position.

The present invention provides a process for producing a compound of formula (I) or a salt, thereof, which process comprises reducing an oxo-group and/or a double bond and/or a nitro group of a compound of formula (II): 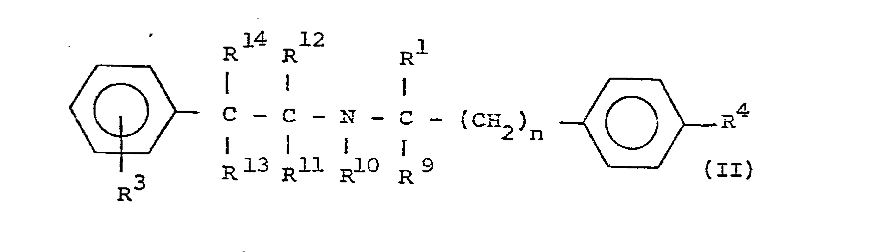
wherein R , R , R and n are as defined in relation formula (I) R⁹ is a group R² as defined in relation to formula (I) or together with R¹⁰ forms a bond;
R¹⁰ is hydrogen or together with R⁹ or R¹¹ forms a bond;
R¹¹ is hydrogen or together with R¹² forms an oxo-group or together with R¹⁰ forms a bond;
R¹² is hydrogen or together with R¹¹ forms an oxo-group;
R¹³ is hydroxyl or together with R¹⁴ forms an oxo-group;
R¹⁴ is hydrogen or together with R¹³forms an oxo-group, provided that there is no more than one oxo-group and no more than one bond represented by any of R⁹ to R¹⁴, and optionally thereafter forming a salt of the compound of formula (I) so formed and/or converting the compound of formula (I) so formed into a further compound of formula (I).

Where there are two or more reducible moieties in the compound of formula (I) these may be reduced separately in any order or simultaneously. When R⁴ is a reducible moiety, this may be reduced before or after a reducible moiety represented by any of R to R¹⁴, or not at all, or, if required and convenient, such a group may be reduced simultaneously with reducible moieties represented by R to R¹⁴. In particular, whenR is nitro it may be reduced to amino and when it is a group 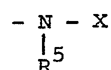 and X is a group 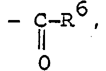 the carbonyl function may be.reduced to methylene.

The aforementioned reductions may be effected by conventional chemical or catalytic methods, such as chemical reduction using lithium aluminium hydride, sodium cyanoborohydride or sodium borohydride or by catalytic hydrogenation using catalysts such as palladium on charcoal, or platinum, for instance, as platinum oxide.

Reduction by sodium borohydride is conveniently effected in a lower alkanolic solvent such as methanol. The reaction is generally carried out at from 0-20^{o}C.

Reduction by lithium aluminium hydride is conveniently effected in a dry, ether solvent such as diethyl ether or tetrahydrofuran at ambient or elevated temperature,

Catalytic reduction is conveniently effected in a conventional hydrogenation solvent such as a lower alkanol, for instance ethanol. The hydrogenation is generally carried out under hydrogen gas at about 1 atmosphere pressure to about 10 atmosphere pressure and at ambient or elevated temperature.

In particular aspects, the present invention provides processes for producing compounds of formula (I) by reducing a compound of formula (IIA): 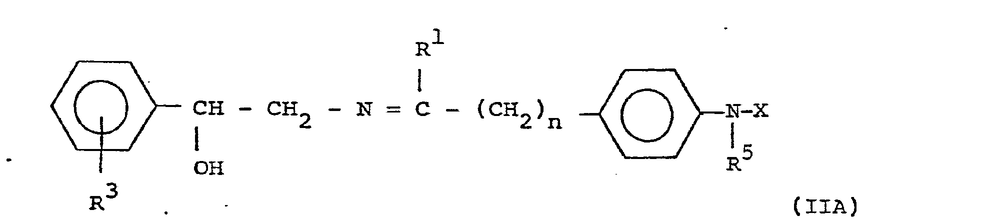 or reducing a compound of formula (IIB): 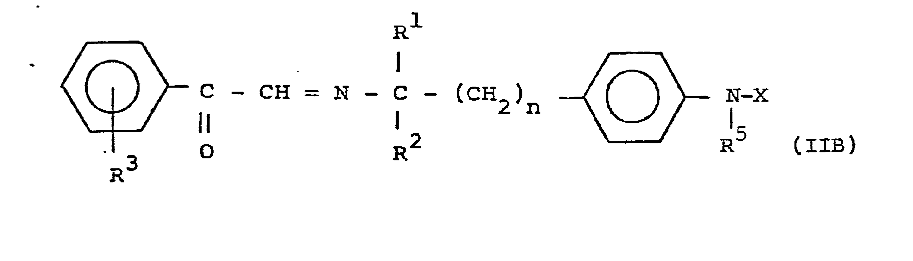 or reducing a compound of formula (IIC): 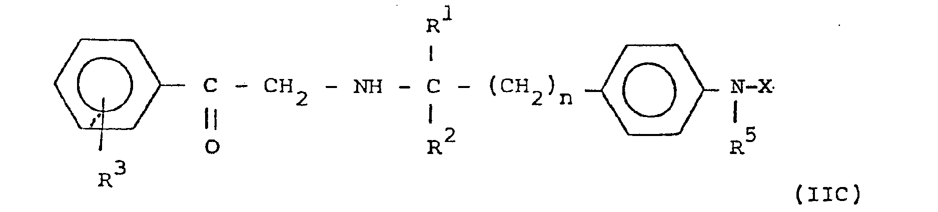 or reducing a compound of formula (IID): 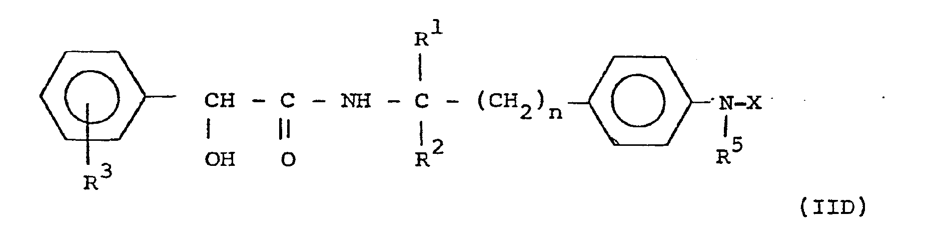 or reducing a compound of formula (IIE): 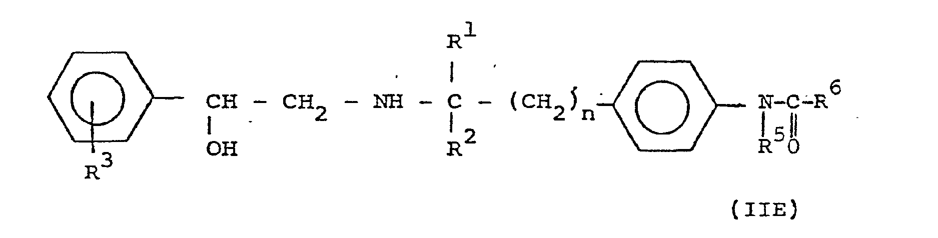 or reducing a compound of formula (IIF) 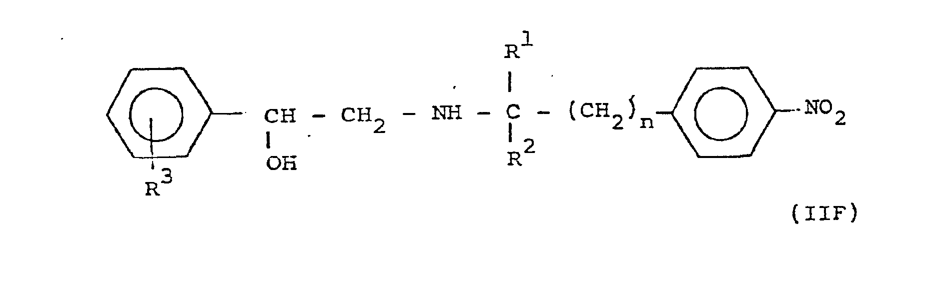 wherein R1, _{R}², _{R}³, R⁵,R⁶, _{X} and n, are as defined in relation to formula (I).

The present invention also provides another process for producing a compound of formula (I) or a salt thereof, which process comprises reacting a compound of formula (III): 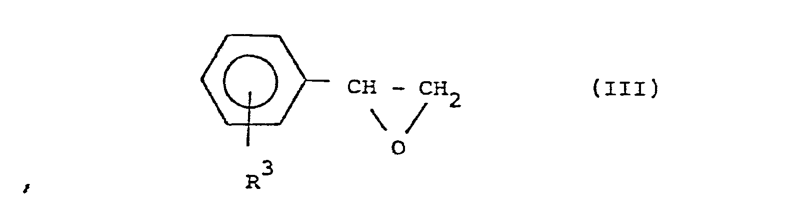 with a compound of formula (IV): 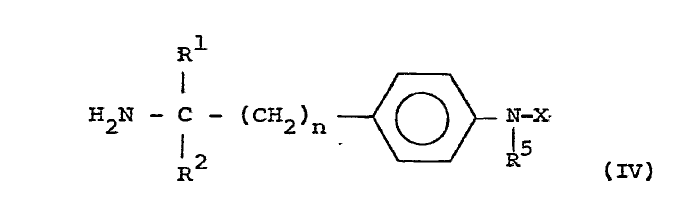 wherein _{R}^{l}, R2, _{R}³, _{R}⁵, _{X} and n are as defined in relation to formula (I), and optionally thereafter forming a salt of the compound of formula (I) so formed.

This reaction is conveniently effected in a solvent such as a lower alkanol, preferably ethanol.

A particular preferred process for producing compounds of formula (I) comprises the reduction of a compound of formula (IIA), especially using sodium borohydride in methanol at ambient temperature.

Compounds of formula (I) may be converted into further compounds of formula (I) by conventional processes. Thus, for example, the compound of formula (I) wherein X is hydrogen may be derivatised to produce further compounds of formula (I). Compounds of formula (I) wherein X is 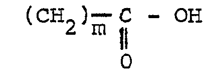 may be converted to the corresponding esters 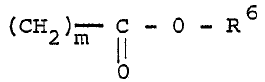 wherein R is C₁₋₆ alkyl. Compounds, wherein X is 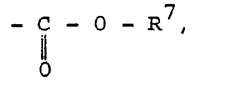 may be reacted with, for instance, primary amines to produce further compounds of formula (I) wherein X is

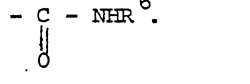

The salts of compounds of formula (I) may be produced by treating the compound of formula (I) with the appropriate acid.

Acyl derivatives of compounds of formula (I) may be produced by conventional methods.

Compounds of formula (I) and salts thereof, produced by the above processes, may be recovered by conventional methods.

Compounds of formula (I) having a single asymmetric carbon atom may, if desired, be separated into individual enantiomers by conventional means, for example by the use of an optically active acid as a resolving agent. Those compounds of formula (I) having two asymmetric carbon atoms may be separated into diastereoisomeric pairs of enantiomers by, for example, fractional cystallisation from a suitable solvent such as ethyl acetate. The pair of enantiomers thus obtained may be separated into individual stereoisomers by conventional means such as by the use of an optically active acid as a resolving agent.

Suitable optically active acids which may be used as resolving agents are described in "Topics in Stereochemistry" Vol 6, Wiley Interscience, 1971, Allinger, N.L. and Eliel, W.L. Eds.

Alternatively any enantiomer of a compound of formula (I) may be obtained by stereospecific synthesis using an optically pure starting material of known configuration.
compounds of formula-(II) may be produced by reacting a compound of formula (V): 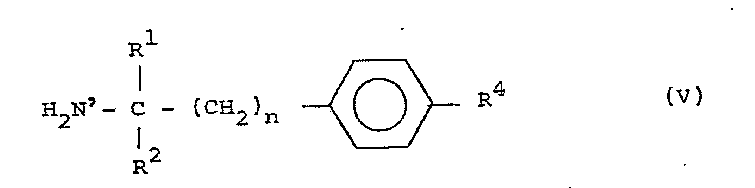 wherein R¹, R², R⁴, and n are as defined in relation to formula. (I).
with a compound of formula (VI): 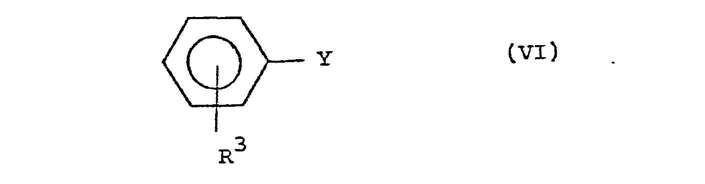 wherein R³ is as defined in relation to formula (I) and Y is a reactive moiety which is capable of reacting with the amine of formula (V) thus forming a compound of formula (II). Typical examples of compounds of formula (VI) are: 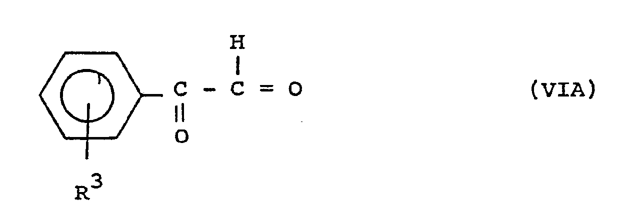 or its hydrate or hemi-acetal of a lower alkanol; 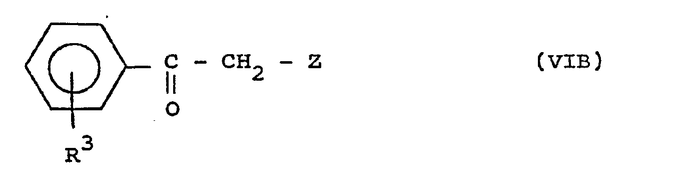 wherein Z is a halogen atom, preferably bromine; 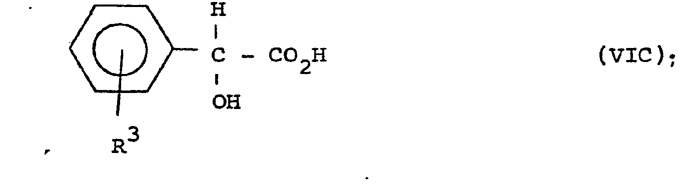 and 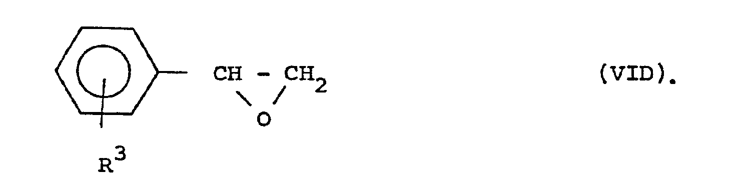 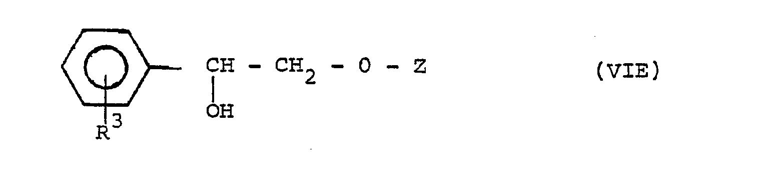 wherein OZ is leaving group, preferably tosyloxy.

Conventional conditions suitable for use with the particular compound of formula (VI) may be used for this reaction. Thus the reaction of a compound of formula (VIA) with a compound of formula (V) is conveniently conducted at elevated temperature under conditions resulting in the removal of the water formed during the reaction. A particularly suitable method is to perform the reaction in a solvent, such as benzene, under reflux and azeotropically to remove the water using a Dean and Stark trap.

The reaction of a compound of formula (VIB) with a compound of formula (V) is conveniently conducted in a polar organic solvent such as acetonitrile or butanone, at an elevated temperature, for instance under reflux.

The reaction of a compound of formula (VIC) with a compound of formula (V) is conveniently conducted under standard peptide formation reaction conditions.

The reaction of a compound of formula (VID) with a compound of formula (V) is conveniently conducted in a solvent such as a lower alkanol, preferably _{'}ethanol.

The reaction of a compound of formula (VIE) with a compound of formula (V) is conveniently conducted in a solvent such as dimethyl sulphoxide at elevated temperature, preferably 50°C for about 3 days.

By using single enantiomers of a compound of formula (V) and a compound of formula (VI) such as the compounds (VIC) or (VID) a stereospecific synthesis of a compound of formula (II) is achieved. This may then be reduced to a Compound of formula (I) without altering the configuration of the two asymmetric carbon atoms. Thus, for example, a compound of formula (V) with the R absolute configuration and a compound of formula (VID) with the R absolute configuration would afford a compound of formula (I) with the RR absolute configuration.

In a modification of the above process for producing compounds of formula (II), the N'-benzyl derivative of a compound of formula (V) may be reacted with a compound of formula (II) is produced and this may be reduced to a compound of formula (I) using a catalytic hydrogenation reaction, especially using palladium on charcoal as catalyst.

Certain compounds of formula (II) may also be produced by reacting a compound of formula (VII): 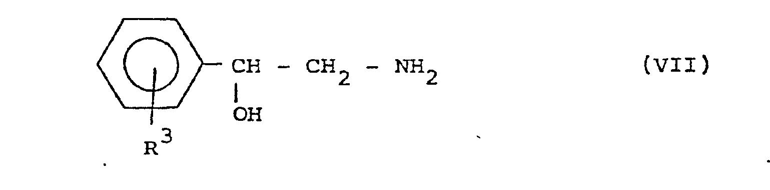 with a compound of the formula (VIII): 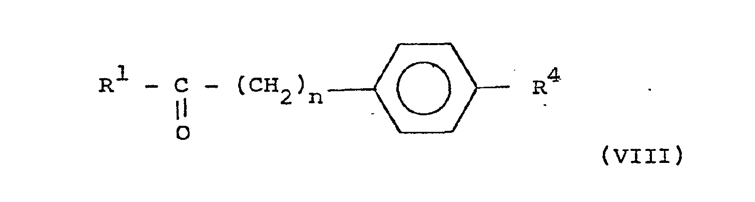 wherein _{R}¹, _{R}³, R and n are as defined in relabion to formula (I).
or a compound of Formula (IX) 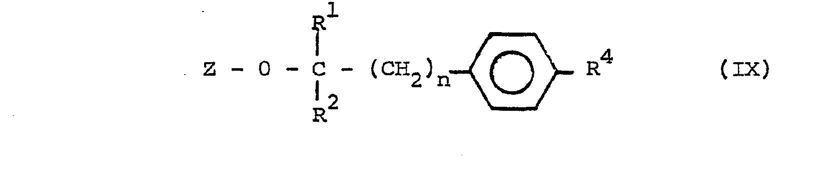 wherein R¹, _{R}², R⁴ and n are as defined in relation to formula (I) and OZ is a leaving group, preferably tosyloxy.

The reaction of a compound of formula (VII) with a compound of formula (VIII) is conveniently effected under conditions which result in the removal of water formed during the reaction. A particularly suitable method is to perform the reaction in a solvent, such as benzene, under reflux and azeotropically to remove the water using a Dean and Stark trap.

The reaction of a compound of formula (VII) with a compound of formula (IX) is conveniently effected in a solvent such as dimethylsulphoxide at elevated temperature, preferably about 50°C for about two to three days.

It is often convenient to prepare the compound of formula (II) and reduce it, in situ, to the desired compound of formula (I) without isolation of the compound of formula (II).

A compound of formula (I) or a pharmaceutically acceptable salt thereof (hereinafter "the drug") may be administered as the pure drug, however, it is preferred that the drug be administered as a pharmaceutical composition also comprising a pharmaceutically acceptable carrier.

Accordingly, the present invention also provides a pharmaceutical composition comprising a compound of formula (I) or a pharmaceutically acceptable salt or in vivo hydrolysable acyl derivative thereof with a pharmaceutically acceptable carrier therefor.

As used herein the terms "pharmaceutical composition" and "pharmaceutically acceptable" embrace compositions and ingredients for both human and veterinary use.

Usually the compositions of the present invention will be adapted for oral administration although compositions for administration by other routes, such as by injection are also envisaged.For treatment of inflammation the compounds would be administered topically.

Particularly suitable compositions for oral administration are unit dosage forms such as tablets and capsules. Other fixed-unit dosage forms, such as powders presented in sachets, may also be used.

In accordance with conventional pharmaceutical practice the carrier may comprise a diluent, binder, filler, disintegrant, wetting agent, lubricant, colourant, flavourant, or the like.

Typical carriers may, therefore, comprise such agents as microcrystalline cellulose, starch, sodium starch glycollate, polyvinylpyrrolidone, polyvinylpolypyrrolidone, magnesium stearate, sodium lauryl sulphate, sucrose and the like.

Most suitably the composition will be provided in unit dose form. Such unit doses will normally comprise 0.1 to 500 mg of the drug, more usually 0.1 to 250 mg and favourably 0.1 to 100 mg.

The present invention further provides a method for treating obesity in humans or domestic mammals, which method comprises administering an effective, non-toxic amount of a compound of formula (I) or a pharmaceutically acceptable salt or in vivo hydrolysable acyl derivative thereof to obese humans or domestic mammals.

Conveniently, the drug may be administered as a pharmaceutical composition as hereinbefore defined, and this forms a particular aspect of the present invention.

In treating obese humans the drug may be taken in doses, such as those described above, one to six times a day in a manner such that the totaly daily dose for a 70 kg adult will generally be about 0.1 to 1000 mg, and more usually about 1 to 500 mg.

In treating obese domestic mammals, especially dogs, the drug may be administered by mouth, usually once or twice a day and at about 0.025 mg/kg to 205 mg/kg, for example 0.1 mg/kg to 2 mg/kg.

The present invention further provides a method for treating hyperglycaemia in humans which method comprises administering an effective, non-toxic amount of a compound of formula (I) or a pharmaceutically acceptable salt or in vivo hydrolysable acyl derivative thereof to hyperglycaemic humans.

Conveniently, the drug may be administered as a pharmaceutical composition as hereinbefore defined, and this forms a particular aspect of the present invention.

The drug may be taken in doses such as those described above for treating obese humans.

The present invention further provides a method for treating inflammation in humans which method comprises administering an effective, non-toxic amount of a compound of formula (I) or a pharmaceutically acceptable salt or in vivo hydrolysable acyl derivative thereof to humans. suffering inflammation.

The invention will now be illustrated with reference to the following Examples, which are not intended to limit the scope in any way.

### EXAMPLE 1

### N-(2-(4-Methylsulphonylaminophenyl)-1-methylethyl]-2-hydroxy-2-phenylethanamine

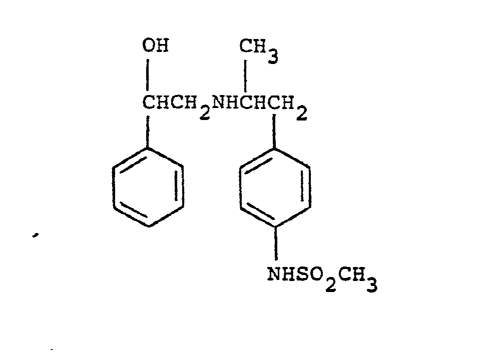

A mixture of phenylethanolamine (1.08 g) and 1-(4-methylsulphonylaminophenyl)propan-2-one (1.8 g) was heated under reflux in benzene (100 ml) with removal of water usina a Dean & Stark trap. After two hours, the benzene was evaporated, the residue dissolved in methanol (50 ml) and the solution cooled inice/water. Sodium borohydride (o.5 g) was added and after ten minutes the solution was evaporated to dryness. The solid residue was partitioned between ethylacetate and water, the organic layer separated, dried and evaporated to give N-[2-(4-methylsulphonylaminophenyl)-1-methylethyl]-2-hydroxy-2-phenylethanamine as an oil. Crystallisation from heptane gave the compound,m.p. 150-156°C, as a 51:49 mixture of diastereoisomers.

¹H n.m.r. γ (DMSO-d₆) 9.06 (3H,d,J=6_{Hz}), 7.0-7.8 (5H,m), 7.1 (3H,s), 5.5 (lH,m), 4.0-5.4 (3H, broad, replaceable by D₂0), 2.8 (4H,m), 2.7 (5H,s).

### EXAMPLE 2

### N-[2-(4-Methylsulphonylaminophenyl)-1-methylethyl]-2-hydroxy-2-(3-trifluoromethylphenyl)ethanamine

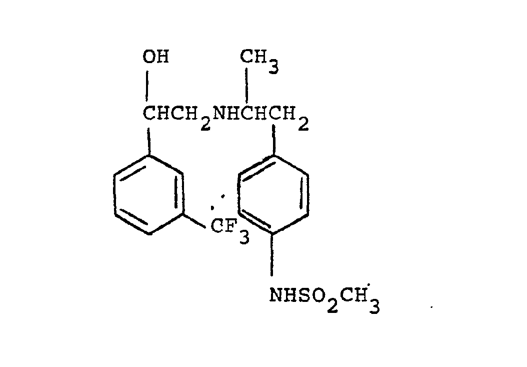

Starting from 2-hydroxy-2-(3-trifluoromethylphenyl) ethanamine (1.63 g) and 1-(4-methylsulphonylaminophenyl) propan-2-one (1.8 g), N-[2-(4-methylsulphonylaminophenyl) -1-methylethyl]-2-hydroxy-2-(3-trifluoromethylphenyl) ethanamine was prepared according to the method of Example 1 and crystallised from hexane/ether, m.p. 53-70°C as a 54:46 mixture of diastereoisomers.

¹_{H} n.m.r.τ(CDCl₃) 8.96 (3_{H},d,J=6_{H}z), 6.8-7.7 (5H,m + 1H replaceable by D₂0), 7.05 (3H,s), 5.25 (lH,m), 4.5-6.0 (2H, broad, replaceable by D₂0), 2.7-3.0 (4H,m), 2.2-2.6 (4H,m).

### EXAMPLE 3

### N-[2-(4-Ethoxycarbonylaminophenyl)-1-methylethyl]-2-hydroxy -2-(3-trifluoromethylphenyl)ethanamine

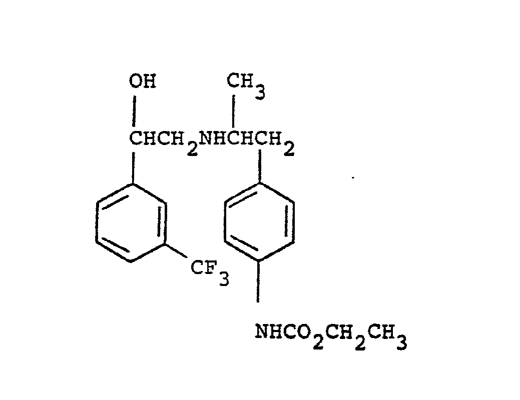

Starting from 2-hydroxy-2-(3-trifluoromethylphenyl) ethanamine (2.69 g) and 1-(4-ethoxycarbonylaminophenyl) propan-2-one (2.9 g), N-[2-(4-ethoxycarbonylaminophenyl) -1-methylethyl]-2-hydroxy-2-(3-trifluoromethylphenyl) ethanamine was prepared according to the method of Example land crystallised from heptane/benzene as a hemihydrate,m.p. 102-104°C,as a mixture of diastereoisomers.

¹ H n.m.r. τ (DMSO-d₆) 9.15 (3H,d,J=6H_{z}), 8.8 (3H,t,J=6Hz), 7.1-7.8 (5_{H},m), 6.4-7.0 (lH, broad, replaceable by D₂0), 5.85 (2H,q,J=6Hz), 5.35 (lH,m), 4.2-4.8 (lH, broad, replaceable by D₂0), 2.9 (2H,dd,J=8_{H}z,J=2Hz), 2.6 (2H,dd, J=8_{H}z, J=2_{H}z), 2.2-2.5 (4H,m), 0.5 (lH,s, replaceable by ^{D}₂⁰).

### EXAMPLE 4

### N-(2-(4-Acetylaminophenyl)-1-methylethyl)-2-hydroxy-2-(3-trifluoromethylphenyl)ethanamine

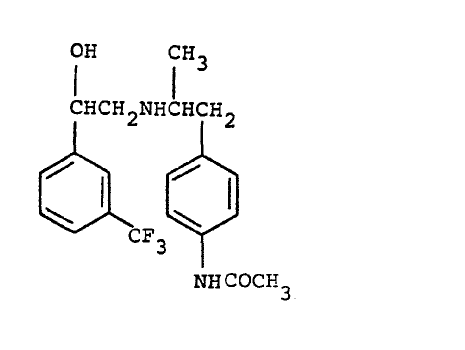

Starting from 2-hydroxy-2-(3-trifluoromethylphenyl) ethanamine (5.8 g) and 1-(4-acetylaminophenyl)propan-2-one (5.4 g),N-[2-(4-a_cetylaminophenyl)-1-methylethyl]-2-hydroxy-2-(3-trifluoromethylphenyl)ethanamine was prepared according to the method of Example 1 and crystallised from dichloromethane, m.p. 85-90°C as a 44:56 mixture of diastereoisomers.

¹H n.m.r. τ (DMSO-d₆) 9.1 (3H,d,J=6Hz), 8.2-8.6 (lH, broad, replaceable by D₂0) 7.95 (3H,s), 7.0-7.6 (SH,m), 6.4-7.0 (lH, broad, replaceable by D₂0), 5.3 (lH,m), 4.5 (lH, broad, replaceable by D₂0), 2.9 (2H,dd, J=8_{Hz}, J=2_{H}z), 2.2-2.65 (6H,m), 0.15 (lH,s, replaceable by ^{D}₂⁰).

### EXAMPLE 5

### N-[2-(4-Methylaminocarbonylaminophenyl)-1-methylethyl]-2-hydroy-2-(3-trifluoromethylphenyl)ethanamine

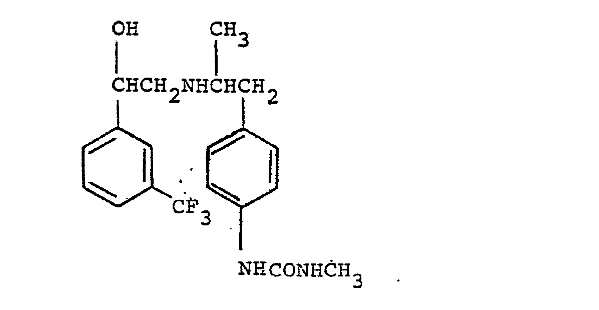

A solution of N-[2-(4-ethoxycarbonylaminophenyl)-1-methylethyl]-2-hydroxy-2-(3-trifluoromethylphenyl)ethanamine (1.0 g) in ethanol (10 ml) was added to a solution of methylamine in ethanol (20 ml) and the mixture heated at 100°C in a screw cap bomb for six hours. After cooling to room temperature, the solution was evaporated, and the residue crystallised from chloroform/methanol affording N- [2-(4-methylaminocarbonylaminophenyl)-1-methylethyl -2-hydroxy-2-(3-trifluoromethylphenyl)ethanamine, m.p. 82-122°C as a 52:48 mixture of diastereoisomers.

¹H n.m.r. τ(DMSO-d₆) 9.1 (3H,d,_{J}=6Hz), 7.35 (3H,d, J=5Hz), 7.0-7.5 (5H,m), 5.7-7.0 (2H, broad, replaceable by D₂0), 5.3 (lH,m), 4.0 (lH,m, slowly replaceable by D₂0), 2.95 (2H,d,J=8Hz), 2.7 (2H,d,J=8Hz), 2.2-2.5 (4H,m), 1.5 (lH,s, replaceable by D₂0)

### EXAMPLE 6

### N-[2-(4-Ethylaminophenyl)-1-methylethyl]-2-hydroxy-2-(3-trifluoromethvlphenvl)ethanamine

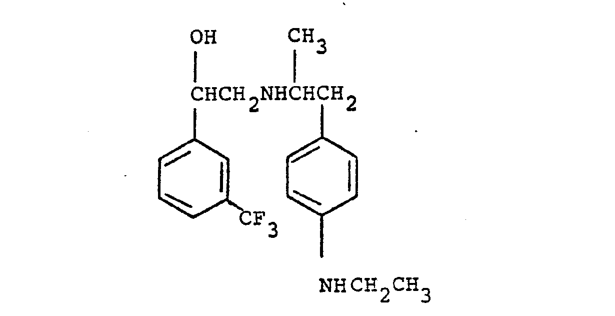

To a slurry of lithium aluminium hydride (1 g) in dry tetrahydrofuran, was added dropwise, a solution of N-[2-(4-acetylaminophenyl)-1-methylethyl]-2-hydroxy-2-(3-trifluoromethylphenyl)ethanamine (1.9 g) in dry tetrahydrofuran (20 ml). The mixture was stirred and heated at reflux for one hour. Water (1 ml), dilute sodium hydroxide solution (1 ml) and water (3 ml) were successively added, the mixture filtered, dried and evaporated. The product was treated with ethereal hydrogen chloride and crystallised from methanol/ether as the dihydrochloride salt of N-[2-(4-eihylaminophenyl)-1-methylethyl]-2-hydroxy -2-(3-trifluoromethylphenyl)ethanamine m.p. 153-173° as a 30:70 mixture of diastereoisomers.

¹H n.m.r. τ(DMSO-d₆) 8.8 (3H,t,J=6Hz), 8.8 (3H,d, J=_{6H}z), 7.2 (lH,m), 6.2-7.0 (6H,m + 3H replaceable by D₂0), 4.7 (lH,m), 2.0-2.7 (8H,m), 0.9 (lH, broad, replaceable by D₂0), 0.2 (1H, broad replaceable by D₂0).

### EXAMPLE 7

### N-[2-(4-Aminophenyl)-1-methylethyl]-2-hydroxy-2-(3-trifluoromethylphenyl) ethanamine

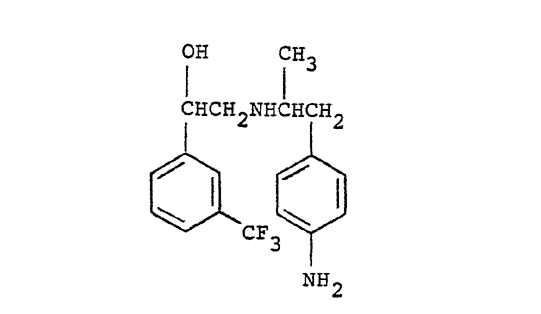

N-[2-(4-Nitrophenyl)-1-methylethyl]-2-hydroxy-2-(3-trifluoromethylphenyl) ethanamine (1.0 g) in ethanol (50 ml) was hydrogenated in the presence of . palladium- charcoal(10% w/w, 0.2 g) at ambient temperature and pressure. When hydrogen uptake ceased the catalyst was filtered and the solvent removed to give N-[2-(4-aminophenyl) -1-methylethyl]-2-hydroxy-2-(3-trifluoromethylphenyl) ethanamine as an oil.

¹H n.m.r. τ(CDCl₃) 8.9 (3H,d,J=6Hz); 6.3-7.4 9H,m, 4 replaceable by D20); 5.35 (lH,m); 3.32 (2H,d, J=8Hz); 3.0 (2H,d,J=8Hz); 2.40 (4H,m)

The product in dry ether was treated with an ethereal solution of hydrogen chloride. The resulting solid was filtered and crystallised from methanol-ethyl acetate to give N-[2-(4-aminophenyl)-1-methylethyl]-2-hydroxy-2-(3-trifluoromethylphenyl) ethanamine dihydrochloride m.p. 250-53°C as a 48:52 mixture of diastereoisomers.

H n.m.r. τ(DMSO-d₆) 8.60 (3H,d,J=6Hz); 6.3-7.6 (7H,m, 2 replaceable by D₂0); 4.80 (1H,m); 2.67 (4H,s); 2.25 (4H,m); 0-1.5 (4H, broad, replaceable by D20)

### EXAMPLE 8

### N-[2-(4-Methoxycarbonylmethylaminophenyl)-1-methylethyl] -2-hydroxy-2-(3-trifluoromethylphenyl) ethanamine

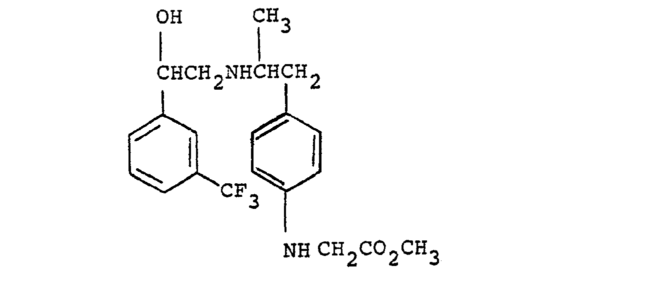

A mixture of 2-hydroxy-2-(3-trifluoromethylphenyl) ethanamine (2.78 g) and 1-(4-methoxycarbonylmethylamino- phenyl) propan-2-one(3.00g) in dry benzene (100 ml) was heated under reflux with removal of water using a Dean and Stark trap. After two hours the benzene was evaporated, the residue dissolved in methanol (50 ml) platinum oxide (50 mg) added and the mixture hydrogenated at ambient temperature and pressure until hydrogen uptake ceased. The solution was filtered through diatomaceous earth, evaporated and the residuechomatographed on silica-gel in methanol dichloromethane mixtures. Crystallisation from methanol- ethylacetate gave N-[2-(4-methoxycarbonylmethylaminophenyl)-1-methylethyl]-2-hydroxy-2-(3-trifluoromethylphenyl) ethanamine m.p. 92-97⁰C as a 64:36 mixture of diastereoisomers.

¹H n.m.r. τ(CDCl₃) 9.0 (3H,d,J=6Hz); 6.80-7.70 (8H,m, 3 replaceable by D₂O); 6.32 (3H,s); 5.85 (2H,s); 5.5 (lH,m); 3.38 (2H,d,J=8Hz); 3.00 (2H,d,J=8_{Hz}); 2.45 (4H,m)

### EXAMPLE 9

### N-[2-(4-Nitrophenyl)-1-methylethyl]-2-hydroxy-2-(3-trifluoromethylphenyl) ethanamine

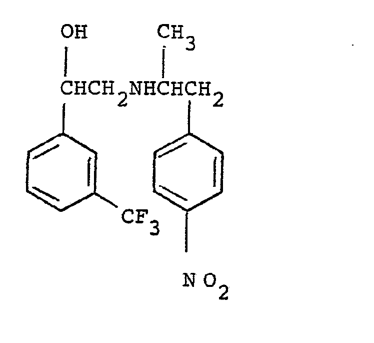

A mixture of 2-hydroxy-2-(3-trifluoromethylphenyl)-ethanamine (6.15 g) and 1-(4-nitrophenyl)propan-2-one (5.37 g) in dry benzene (80 ml) was heated under reflux in a Dean and Stark apparatus. After two hours the benzene was evaporated, the residue dissolved in methanol (150 ml) and the solution cooled in iced water. Sodium borohydride (5 g) was added portionwise with stirring and on completion of the addition the mixture was stirred at room temperature for two hours. Water (200 ml) was added, extracted with dichloromethane, the organic layers dried (magnesium sulphate) and evaporated. The residue was eluted from silica-gel in methanol-dichloromethane mixtures to give N-[2-(4- itrophenyl)-1-methylethyl]-2-hydroxy-2-(3-trifluoromethylphenyl) ethanamine m.p. 70-75 C as a 52:48 mixture of diastereoisomers.

1H n.m.r. π(CDCl₃) 8.95 (3H,d,J=4_{H}z); 6.8-8.0 (7H,m,2H replaceable by D20); 5.38 (lH,m); 2.73 (2H,d, J=6_{H}z; 2.3-2.6 (4H,m); 1.87 (2H,d,J=6Hz)

### EXAMPLE 10

### N-[2-(4-Dimethylaminophenyl]-1-methylethyl]-2-hydroxy-2-(3-trifluoromethylphenvl)ethanamine

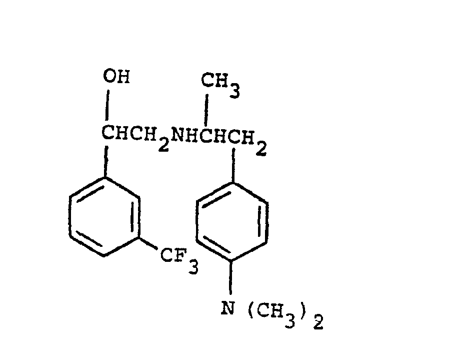

Starting from 2-hydroxy-2-(3-trifluoromethylphenyl) ethanamine (4.7 g) and 1-(4-dimethylaminophenyl)propan-2-one (4.01 g), N-[2-(4-dimethylaminophenyl]-1-methylethyl]-2-hydroxy-2-(3-trifluoromethyl-phenyl)ethanamine was prepared according to the method of Example 1 and crystallised from hexane, mp 92-105°C as a 32:68 mixture of diastereoisomers.

¹H n.m.r. τ(CDCl₃) 8.95 (3H,d,J=6Hz), 6.95-7.6 (5H,m + 2H replaceable by D₂0), 7.10 (6H,s), 5.40 (lH,m), 3.32 (2H,dd,J=8Hz,J=2Hz), 3.01 (2H,dd,J=8_{Hz}, J=2_{H}z), 2.5 (4H,m).

### EXAMPLE 11

### N- [2-(4-(1-Pyrrolidyl)phenyl)-1-methylethyl] -2-hydroxy -2-(3-trifluoromethylphenyl)ethanamine

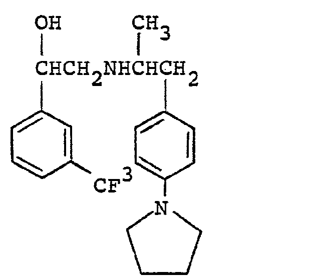

Starting from 2-hydroxy-2-(3-trifluoromethylphenyl)ethanamine (0.7g) and 1-(4-(1-pyrrolidyl)phenyl)propan-2-one (0.7g), N- [2-(4-(1-pyrrolidyl)phenyl)-1-methylethyl]-2-hydroxy -2-(3-trifluoromethylphenyl)ethanamine m.p. 103-117°C was prepared according to the method of Example 1 and crystallised from hexane as a 38:62 mixture of diastereoisomers. ¹H nmr τ(CDCl₃)

8.9 (3H,d,J=6HZ), 8.0 (4H,m), 6.9-7.7 (5H,m + 2H replaceable by _{D2}0), 6.7 (4H,m), 5.4 (lH,m), 3.5 (2H,dd,J=8Hz, J=2H_{z}), 3.0 (2H,dd,J=8H_{z}, J=2H_{z}), 2.3-2.7 (4H,m).

### EXAMPLE 12

### N-[2-(4-(4-Morpholino)phenyl)-1-methylethyl]-2-hydroxy -2-(3-trifluoromethylphenyl)ethanamine

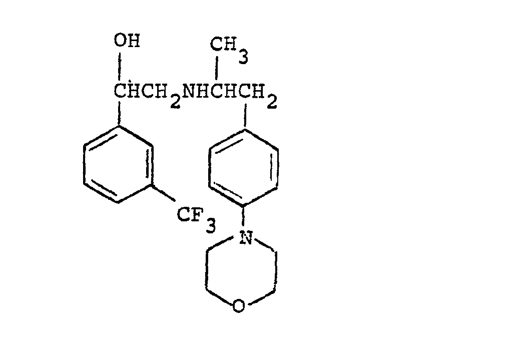

Starting from 2-hydroxy-2-(3-trifluoromethylphenyl)ethanamine (2.81g) and 1-[4-(4-morpholino)phenyl]propan-2-one (3g), N-[2-(4-(4-morpholino)phenyl)-1-methylethyl]-2-hydroxy -2-(3-trifluoromethylphenyl)ethanamine.m.p. 92-112°C was prepared according to the method of Example 1 and crystallised from hexane as a 50:50 mixture of diastereoisomers.

### 1_{H} nmr τ(CDCl₃)

8.9 (3H,d,J=8H_{z}), 6.75-7.50 (10H,m, + 1H replaceable by D₂0), 6.0-6.3 (4H,m), 5.25-5.55 (lH,m), 3.2 (2H,dd, J=8H_{z}, J=2H_{z}), 2.9 (2H,dd,J=8H_{z}, J=2Hz), 2.3-2.6 (4H,m).

### EXAMPLE 13

### N-[2-(4-(1-(4-Carboethoxy)piperidyl)phenyl)-1-methylethyl]-2-hydroxy-2-(3-trifluoromethylphenyl)ethanamine

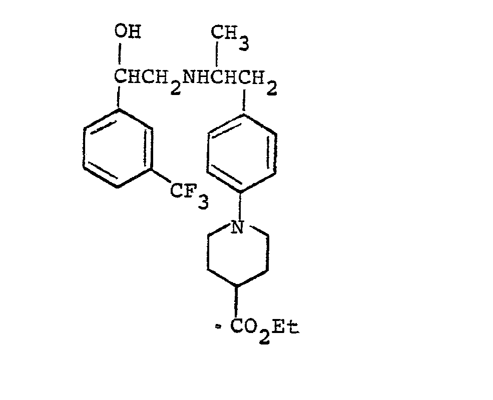

Starting from 2-hydroxy-2-(3-trifluoromethylphenyl)-ethanamine (4.1g) and 1-[4-(1-(4-carboethoxy)piperidyl) phenyllpropan-2-one (5.78g), N-[2-(4-(1-(4-carboethoxy) piperidyl)phenyl)-1-methylethyl]-2-hydroxy-2-(3-trifluoromethylphenyl)ethanamine m.p. 75-79⁰C was prepared according to the method of Example 1 as a 50:50 mixture of diastereoisomers.

### ¹_{H} nmr τ(CDCl₃)

8.9 (3H,d,J=8H_{z}), 8.75 (3H,t,J=7H_{z}), 7.9-8.45 (4H,m), 6.9-7.75 (8H,m + 2H replaceable by D₂0), 6.25-6.6 (2H,m), 5.9 (2H,q,J=7H_{z}), 5.25-5.55 (lH,m), 2.9-3.3 (4H,m), 2.3-2.65 (4H,m).

### EXAMPLE 14

### N-[2-(4-(1-(4-N'-Methylcarboxamido)piperidyl)phenyl)-1-methylethyl]-2-hydroxy-2-(3-trifluoromethylphenyl)ethanamine

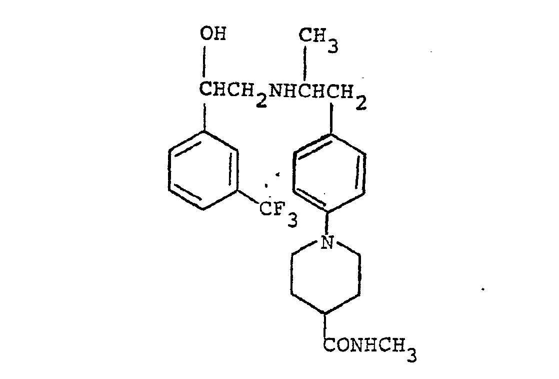

N-[2-(4-(1-(4-carboethoxy)piperidyl)phenyl-1-methylethyl]-2- hydroxy-2-(3-trifluoromethylphenyl)ethanamine (1.5g) was dissolved in ethanol containing methylamine, and heated at 100°C in a screw cap bomb for 5 hours. The ethanol was evaporated and the residue crystallised for diethyl ether to afford N-[2-(4-(1-(4-N'methylcarboxamido)piperidyl) phenyl)-1-methylethyl]-2-hydroxy-2-(3-trifluoromethylphenyl) ethanamine m.p. 130-144°C, as the hemihydrate.

### ¹H nmr τ(CDCl₃)

8.8 (3H,d,J=8H_{z}), 7.9-8.2 (4H,m), 6.7-7.5 (7H,m + 3H,d, J=4H_{z}), 6.0-6.6 (3H,m), 5.7 (3H, broad singlet, replaceable by D₂0), 5.1 (lH,m), 4.3 (lH,bd, replaceable by D₂0), 2.8-3.2 (4H,m), 2.2-2.6 (4H,m).

### DESCRIPTION 1

### 1-(4-Methylsulphonylaminophenyl)propan-2-one

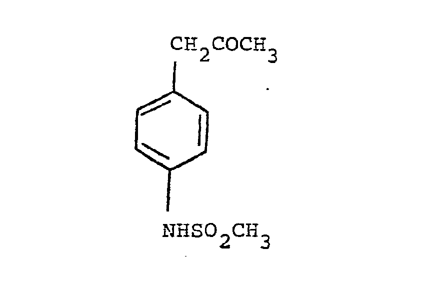

To a stirred solution of 1-(4-aminophenyl)prcpan -2-one ethylene ketal (2 g) in dry pyridine (20 ml), was added dropwise, methanesulphonyl chloride (1.48 g). After four hours at room temperature the mixture was poured onto ice/water and the product extracted into chloroform. The chloroform extracts were evaporated to dryness, the residue was dissolved in acetone (100 ml) and hydrochloric acid (2M,2 ml), and the solution stirred at room temperature for sixteen hours. The solution was evaporated to remove the acetone, and the residue extracted with chloroform. Drying (magnesium sulphate) and removal of solvent gave 1-(4 m ethylsulphonylaminophenyl)propan-2- one, as an oil.

¹H n.m.r. τ(CDCl₃) 7.8 (3H,s), 7.0 (3H,s), 6.3 (2H,s), 2.85 (4H,s), 2.55 (lH,s, replaceable by D₂Q).

### DESCRIPTION 2

### 1-(4-Ethoxycarbonylaminophenyl)propan-2-one

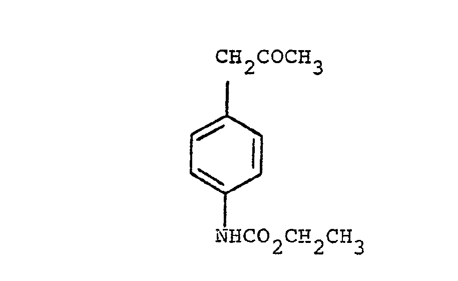

1-(4-Ethoxycarbonylaminophenyl)propan-2-one was prepared according to the method of Description 1 but replacing methanesulphonylchloride with ethylchloroformate.

1H n.m.r. τ(CDCl₃) 8.7 (3H,t,J=6Hz), 7.8 (3H,s), 6.3 (2H,s), 5.7 (2H,q,J=6Hz), 2.3-3.0 (4H,m + 1H replaceable by ^{D}₂⁰).

### DESCRIPTION 3

### 1-(4-Acetylaminophenyl)propan-2-one

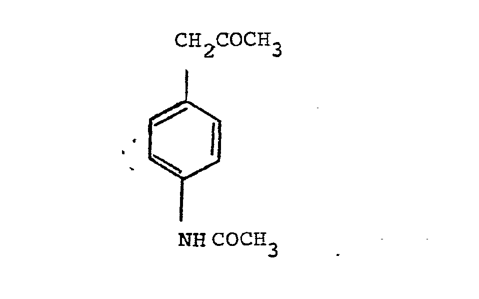

To a solution of 1-(4-aminophenyl)propan-2-one ethylene ketal (6 g) in toluene, was added acetic anhydride (20 ml). The solution was stirred and heated under reflux for 2 hours, cooled, the solvent removed and the residue dissolved in acetone (100 ml) and hydrochloric acid (2M, 10 ml). After sixteen hours at room temperature the solution was neutralised by addition of sodium bicarbonate solution and the acetone evaporated. The aqueous residue was extracted with ethylacetate, the organic phase dried (magnesium sulphate)and evaporated to give 1-(4-acetylaminophenyl)propan-2-one.

¹H n.m.r. α(CDCl₃) 7.8 (6H,s), 6.3 (2H,s), 2.9 (2H,d,J=6H_{z}), 2.55 (2H,d,J=6_{H}Z) 2.0 (lH, broad, replaceable by D₂⁰)

### DESCRIPTION 4

### 1-(4-Nitrophenyl)propan-2-one-ethylene ketal

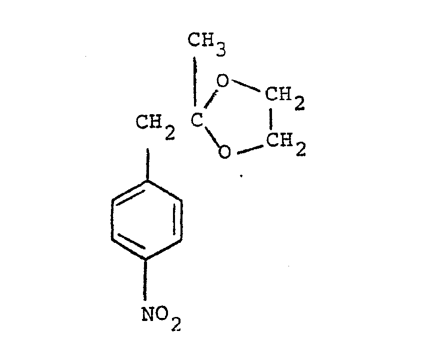

To a stirred solution of 4-nitrophenylpropanone (10 g) in dry benzene (100 ml) was added ethylene glycol (10 ml) andp-toluenesulphonic acid (100 mg). The mixture was boiled under reflux in a Dean and Stark apparatus until water evolution ceased. The mixture was cooled to room temperature, washed with water, dried(magnesium sulphate)and the solvent removed under reduced pressure to give 1-(4-nitrophenyl)propan-2-one-ethylene ketal as a red oil.

1H n.m.r. τ(CDCl₃) 8.7 (3H,s); 6.9 (2H,s); 6.0-6.35 (4H,m); 2.55 (2H,d,J=8Hz); 1.72 (2H,d,J=8_{H}z).

### DESCRIPTION 5

### 1-(4-Aminophenyl)propan-2-one ethylene ketal

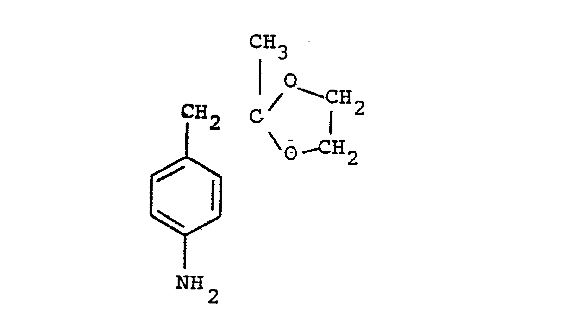

1-(4-Nitrophenyl)propan-2-one ethylene ketal (5 g) in ethanol (70 ml) was hydrogenated in the presence of palladium on charcoal (10% w/w) at 60 psi and room temperature. When hydrogen uptake had ceased the catalyst was filtered off and the solvent removed under reduced pressure to give 1-(4-aminophenyl)propan-2-one ethylene ketal as a red oil.

¹H n.m.r. τ(CDCl₃) 8.71 (3H,s); 7.27 (2H,s); 6.65 (2H, broad, replaceable by D₂O); 6.15 (4H,m); 3.43 (2H,d, J=8Hz); 2.96 (2H,d,J=8Hz)

### DESCRIPTION 6

### 1-(4-Methoxycarbonylmethylaminophenyl)propan-2-one

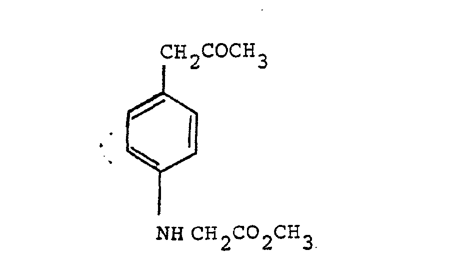

Methyl bromoacetate (3.06 g) was added to a solution of 1-(4-aminophenyl)propan-2-one ethyleneketal (3.86 g) in hexamethylphosphoramide (20 ml) and the mixture was stirred at 110°C for two hours. After cooling, water (250 ml) was added and the solution extracted with ether. The combined organic extracts were washed with water, dried (magnesium sulphate) and evaporated to dryness. The residue was dissolved in methanol (40 ml), hydrochloric acid (2M, 10 ml) was added and the solution was stirred at room temperature for two hours. The solution was basified with saturated sodium bicarbonate solution, extracted with ether, the organic extracts dried (magnesium sulphate) and evaporated to give 1-(4-methoxycarbonylmethyl- aminophenyl)propan-2-one.

¹H n.m.r. τ(CDCl₃) 7.95 (3H,s); 6.45 (2H,s); 6.23 (3H,s); 6.10 (2H,s); 3.45 (2H,d,J=8Hz); 2.93 (2H,d,J=8Hz)

### DESCRIPTION 7

### 1-(4-Dimethylaminophenyl)-2-nitroprop-1-ene

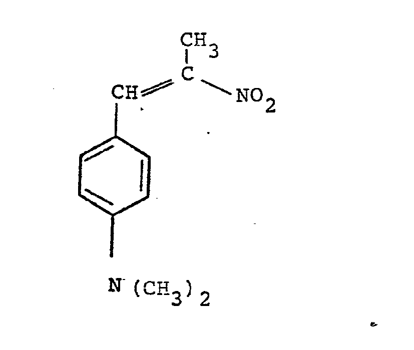

A mixture of 4-dimethylaminobenzaldehyde (18.3 g) and ammonium acetate (9.66 g) in nitroethane (75 ml) was heated to boiling for 1 hour. On cooling the product separated as a deep red solid (10 g).

¹H n.m.r. π(CDCl₃) 7.5 (3H,s), 6.9 (6H,s), 7.26 (2H,d,J=8Hz), 2.6 (2H,d,J=8_{H}z), 1.98 (lH,s).

### DESCRIPTION 8

### 1 - (4-Dimethylaminophenyl)propan-2-one

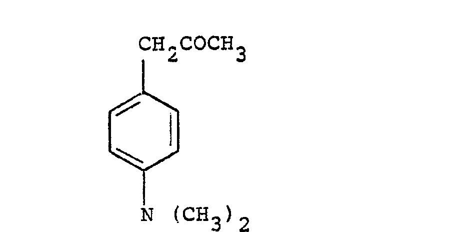

To a stirred mixture of 1-(4-dimethylaminophenyl)-2-nitro-prop-1-ene (22 g) and iron (22 g) in tetrahydrofuran (200 ml) was added concentrated hydrochloric acid (130 ml) at reflux over a period of 1 hour. Heating was continued for a further 1 hour, the solution cooled, filtered through diatomaceous earth, and evaporated. The residue was neutralised by addition of sodium hydroxide solution (2M) and extracted with ether. The ether extract was washed successively with sodium bicarbonate solution (1.2M), water and dried (magne sium sulphate). Evaporation gave an oil which was distilled at reduced pressure to give 1-(4-dimethylaminophenyl)propan-2-one (7.6 g) b.p. 110-113°C (0.4 mm).

¹H n.m.r. τ(CDCl₃) 7.95 (3H,s), 7.12 (6H,s), 6.5 (2H,s), 3.35 (2H,d,J=8Hz), 2.95 (2H,d,J=8Hz)

### DESCRIPTION 9

### 4-(1-Pyrrolidyl)benzaldehyde

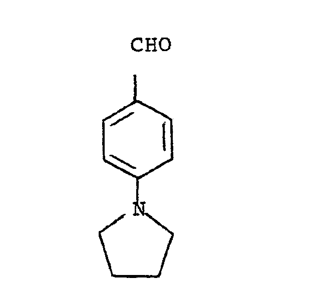

A mixture of 4-fluorobenzaldehyde (14.8g) pyrrolidine (9.94g) and anhydrous pottassium carbonate (16.56g) in dimethyl sulphoxide (50ml) was headed at 95°C for 6 hours. The reaction mixture was cooled, poured in to ice-water (300ml) and the precipitated solid isolated by filtration. The solid was crystallised from heptane, to give the title compound m.p. 80-84°C.

### ¹H nmr τ(CDCl₃)

7.9 (4H,m), 6.6 (4H,m), 3.4 (2H,d,J=8H_{z}) 2.2 (2H,d,J=8H_{z}), 0.2 (lH,s) .

### DESCRIPTION 10

### 1-[4-(l-Pyrrolidyl)phenyl]-2-nitropro-1-ene

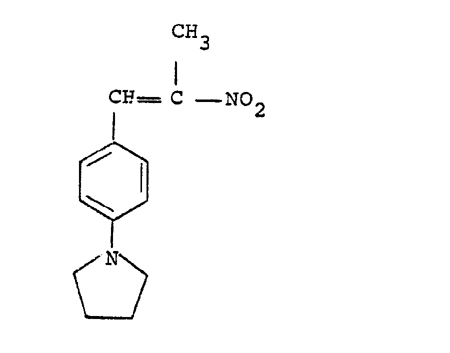

The title compound was prepared as in Description 7 using 4-(1-pyrrolidyl)benzaldehyde instead of 4-dimethylaminobenzaldehyde.

### ¹H nmr τ(CDCl₃)

7.95 (4H,m), 7.55 (3H,s), 6.68 (4H,m), 3.45 (2H,d,J=8H_{z}), 2.65 (2H,d,J=8H_{z}), 1.95 (lH,s).

### DESCRIPTION 11

### 1-[4-(1-Pyrrolidyl)phenyl]propan-2-one

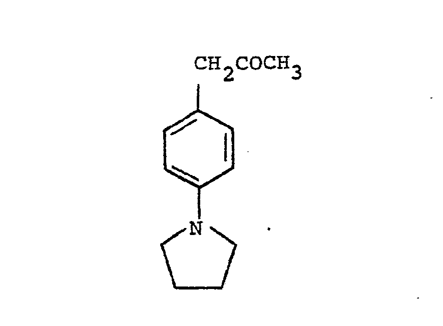

The title compound was prepared as in Description 8, using 1-[4-(1-pyrrolidyl)phenyl]-2-nitroprop-1-ene, instead of 1-(4-dimethylaminophenyl)-2-nitroprop-1-ener as an oil, b.p. 60-65°C/0.5mm Hg.

### ¹_{H} nmr τ(CDCl₃)

8.0 (4H,m), 7.9 (3H,s), 6.7 (4H,m), 6.4 (2H,s), 3.5 (2H,d, J=8_{Hz}), 2.95 (2H,d, J=8H_{z}).

### DESCRIPTION 12

### 4-(4-Morpholino)benzaldehyde

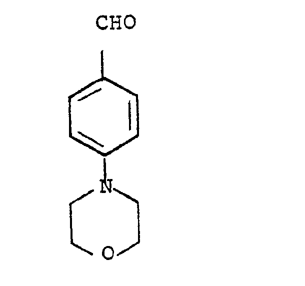

The title compound was prepared as in Description 9 using morpholine instead of pyrrolidine and crystallised from ethanol m.p. 61-63⁰C.

### 1H nmr τ(CDCl₃)

6.7 (4_{H},m), 6.2 (4H,m), 3.1 (2H,d,J=8H_{z}), 2.25 (2H,d, J=8H_{z}), 0.2 (lH,s).

### DESCRIPTION 13

### 1-[4-(4-Morpholino)phenyl]-2-nitropro-1-ene

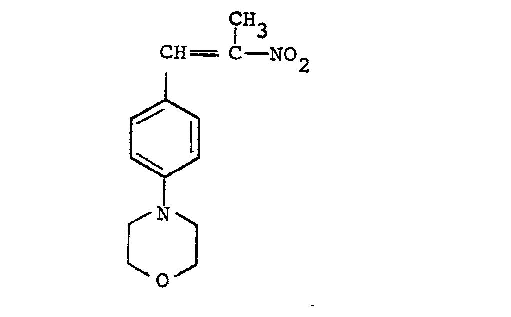

A mixture of 1-(4-morpholino)benzaldehyde (10g) and n-butylamine (7.0ml) in benzene, was heated under reflux for 3 hours with the water formed removed by a Dean and Stark trap. The solution was cooled, evaporated and the residue dissolved in glacial acetic acid (20ml). Nitroethane (9.5m1) was then added and the mixture stirred and heated at 100°C for 1 hour. The reaction mixture was cooled, evaporated to dryness and the solid residue crystallised from ethanol.

### 1_{H} nmr τ (CDCl₃)

7.5 (3H,s), 6.7 (4H,m), 6.2 (4H,m), 3.1 (2H,d,J=8_{Hz}), 2.5 (2H,d,J=8H_{z}), 1.95 (1H,s).

### DESCRIPTION 14

### 1-[4-(4-morpholino)phenyl]propan-2-one

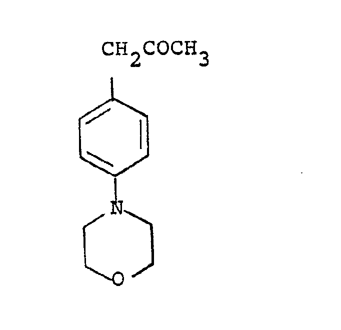

The title compound was prepared as in Description 8,using 1-[4-(4-morpholino)phenyl]-2-nitroprop-1-ene instead of 1-(4-dimethylamino)-2-nitroprop-1-ene, as an oil b.p. 149-150°C /0.5mm Hg.

### ¹H nmr τ(CDCl₃)

7.9 (3H,s), 6.9 (4H,m), 6.4 (2H,s), 6.2 (4H,m),
2.7-3.3 (4H,m).

### DESCRIPTION 15

### 4-[1-(4-Carboethoxy)piperidyl]benzaldehyde

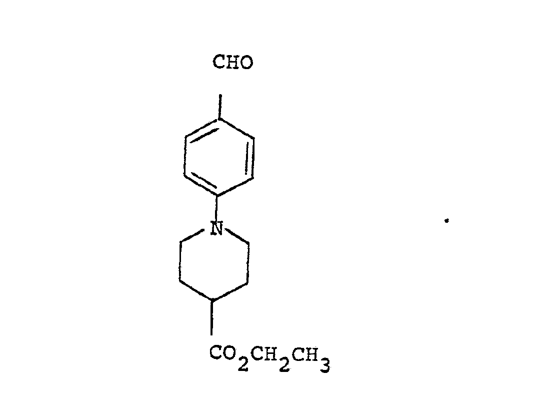

The title compound was prepared as in Description 9 from ethylisonipecotate (29.4g) and 4-fluorobenzaldehyde (20g).

### ¹H nmr τ(CDCl₃)

8.75 (3H,t,J=8H_{z}), 6.7-8.5 (8H,m), 5.8-6.3 (lH,m), 5.8 (2H,q,J=8H_{z}), 3.1 (2H,d,J=8H_{z}), 2.3 (2H,d,J=8H_{z}), 0.3 (lH,s).

### DESCRIPTION 16

### 1-[4-(1-(4-Carboethoxy)piperidyl)phenyl]-2-nitroprop-1-ene

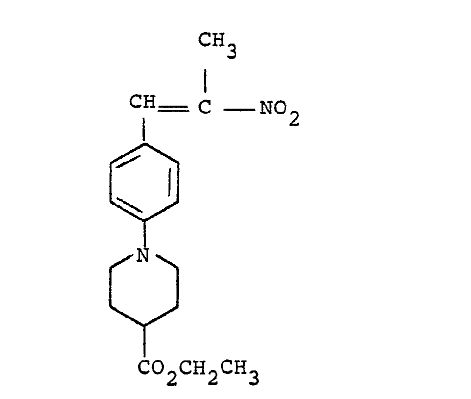

The title compound was prepared as in Description 7 using 4-[1-(4-carboethoxy)piperidyl]benzaldehyde instead of 4-dimethylaminobenzaldehyde.

### ¹_{H} nmr τ(CDCl₃)

8.8 (3H,t,J=6H_{z}), 7.8-8.4 (4H,m), 7.5 (3H,s), 6.7-7.3 (4H,m), 6.0-6.5 (lH,m), 5.9 (2H,q,J=6H_{z}), 3.2 (2H,d,J=8H_{z}), 2.6 (2H,d,J=8H_{z}), 2.0 (lH,s).

### DESCRIPTION 17

### 1-[4-(1-(4-Carboethoxy)piperidyl)phenyl]propan-2-one

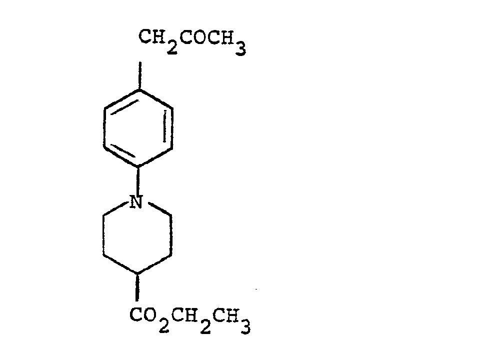

The title compound was prepared as in Description 8 using 1-[4-(1-(4-carboethoxypiperidyl)phenyl]-2-nitroprop-1-ene instead of 1-(4-dimethylaminophenyl)-2-nitroprop-1-ene.

### ¹_{H} nmr τ(CDCl₃)

8.75 (3H,t,J=6H_{z}), 7.0-8.3 (8H,m), 7.88 (3H,s), 6.2-6.6 (lH,m), 6.4 (2H,s), 5.8 (2H,q,J=6H_{Z}), 2.7-3.3 (4H,m).

### PHARMACOLOGICAL DATA

### Effect on energy expenditure

The effect of the compounds on the energy expenditure of mice was demonstrated by means of the following procedure:
Female CFLP mice each weighing approximately 24 g, were given food and water ad lib before and during the experiment. The compounds were dissolved in water by addition of one mole of hydrochloric acid per mole of compound and these solutions were administered orally to each of 12 mice. A further 12 mice were dosed orally with water. The mice were placed in boxes through which air was drawn and the oxygen content of the air leaving the boxes was measured. The energy expenditure of the mice was calculated for 21 hours after dosing from the volume of air leaving the boxes and its oxygen content, following the principles described by J.B. de V. Weir, J. Ph^{y}siol. (London) 109, 1-9, (1949). The food intake of the mice was measured over this same period of 21 hours. The results are expressed as a percentage of the mean food intake or rate of energy expenditure of the mice dosed with water. Results are given in Table I.

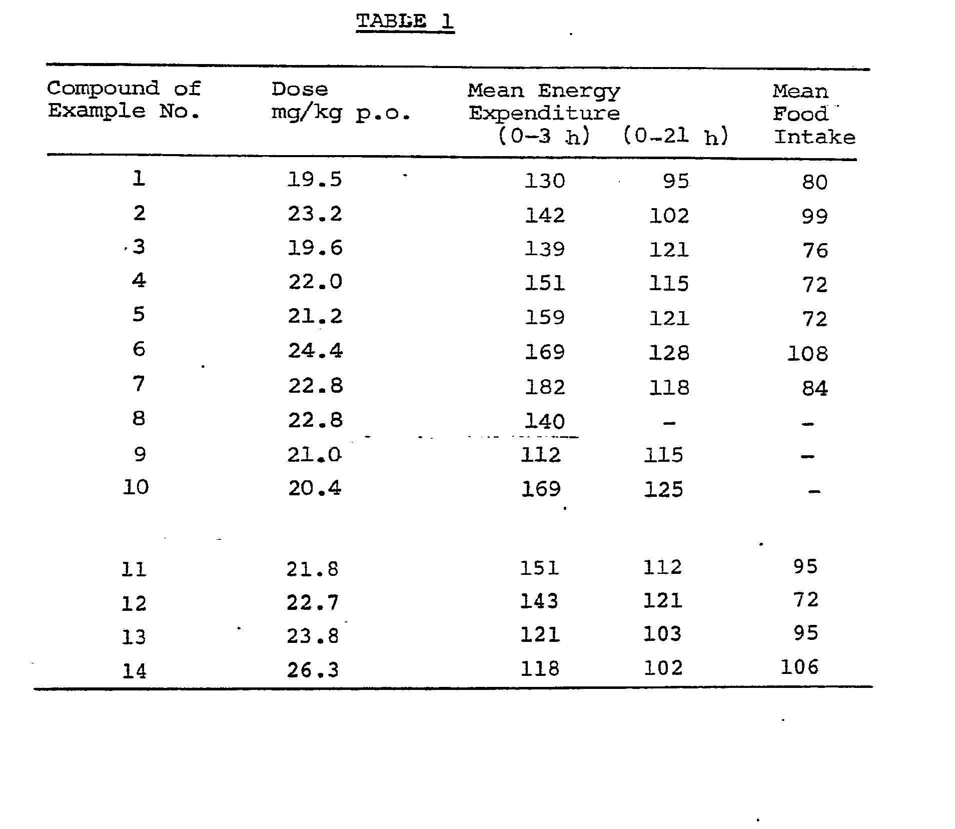

### ii) Cardiac Activity

Rat hearts were perfused by the Langendorff procedure. Hearts were dissected free within 30 seconds of death and reverse perfused via the aorta and coronary vessels with Krebs-Ringer bicarbonate solution (pH 7.4, 37°C) gassed with 95% oxygen: 5% carbon dioxide at a flow rate between 8-12 cm³/minute. Responses were observed after injection of drug dissolved in isotonic saline into the perfusion media. Heart rate and tension were displayed on an Ormed MX2P recorder via a tension transducer and heart ratemeter.

Results are given in Table 2, expressed as a percentage of the maximum response due to salbutamol.

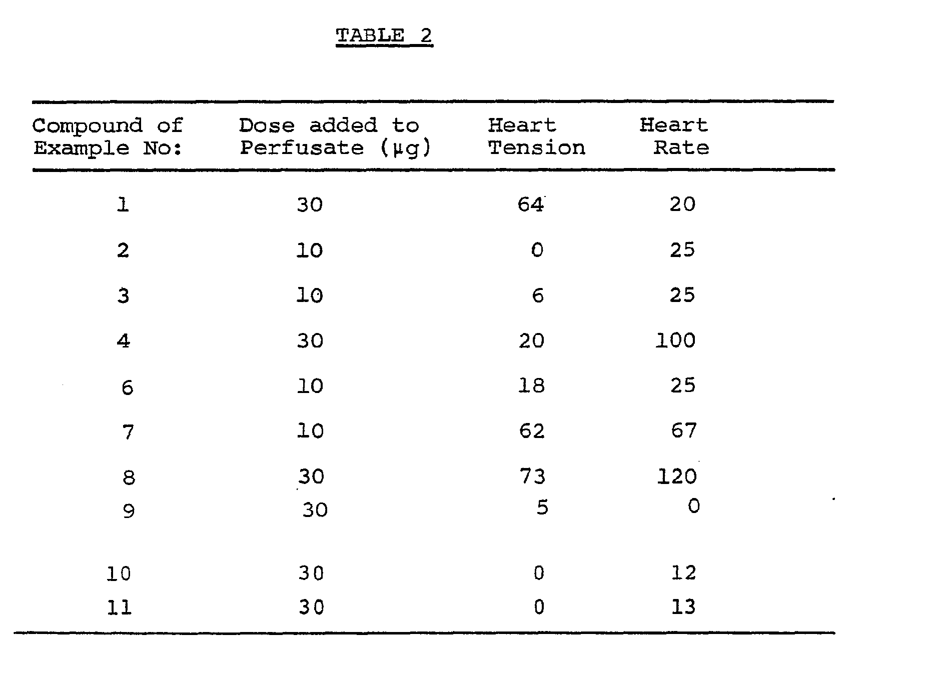

### iii) Hypoglycaemic Activity

Female CFLP mice, weighing approximately 25 g, were fasted for 24 hours prior to the study. The compounds under study were administered orally as an aqueous solution to each of 8 mice. 30 minutes later a blood sample (20 µℓ) was obtained from the tail for the analysis of blood glucose. Immediately after taking this blood sample, glucose (1 g/kg body weight) was administered subcutaneously to each mouse. 8 mice were given water as a control. Blood samples were then obtained from each mouse at 30-minute intervals for 120 minutes.

Compounds that produced a significant (P<0.05) reduction of blood glucose, compared with control mice given water, at any time interval , were considered active. The area under the blood glucose curve over the 2 hour period after the administration of the glucose was calculated for each compound and compared with the value for control animals. Results are given in Table 3.

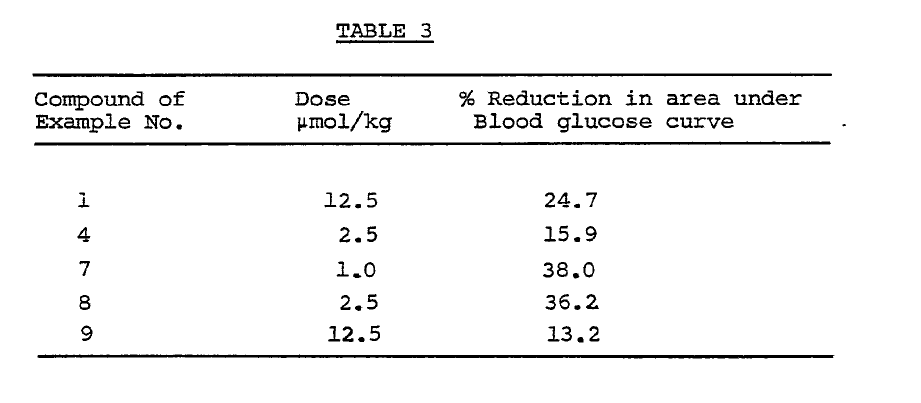

### iv) Platelet Aggregation Inhibition Activity

Male CFLP mice (ca 20 g, n=8) were dosed orally with compound or vehicle (controls) after an overnight fast. Two hours later each mouse received an intravenous dose of collagen (400 µg/kg, pH 6-6.6). Exactly 30 sec after injection of collagen, each mouse was placed in a chamber of C0₂ until respiration ceased. Blood platelet concentration was determined (Ultra-Flo 100 whole blood platelet counter, Clay Adams) in blood samples (3 µl) taken immediately from the inferior vena cava. Each concentration was expressed as a per cent of that obtained in a tail blood sample taken immediately before injection of collagen. Results are given in Table 4.

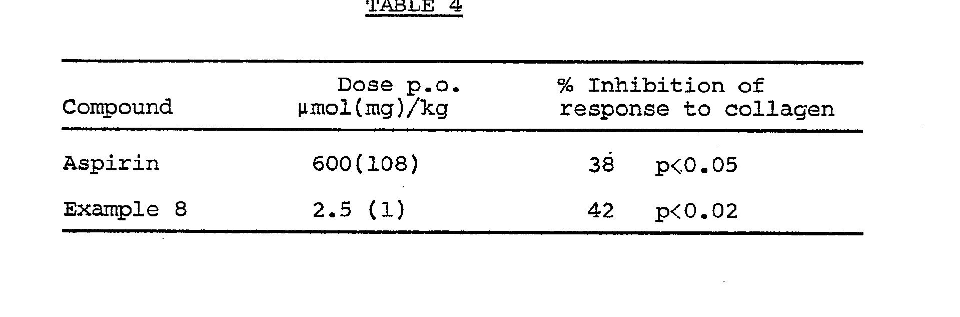

### v) Anti-obesity activity

The compounds were administered by oral gavage in water or carboxymethyl-cellulose suspension to genetically obese mice daily for 28 days. At the end of the time the carcass composition was determined. The results obtained were as follows:

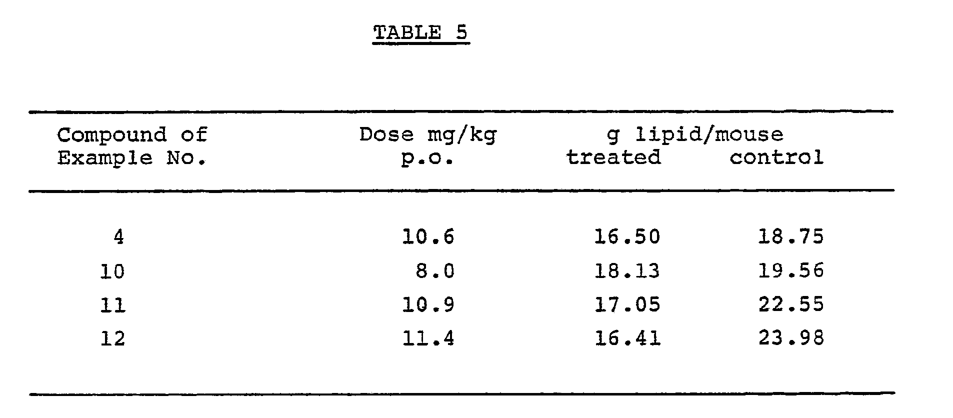

### vi) Toxicity

No toxic effects were observed during the above experiments.

### CORRIGENDA

### The following amendments should be effected in the Description and Examples

Page 7 last line after "methanol" add "or ethanol"
Page 10 after formula (III) add "or a compound of formula (VIE) as defined hereinbelow"
Page 10 line 15 after "methanol" add "or ethanol"
Page 14 line 28 after "(II)" add "or (I)"
Page 15 between lines 5 and 6 add "formula (VIB). In this case the N-benzyl derivative of a compound of"
Page 19 between lines 17 and 18 insert
   "The present invention further provides a method for inhibiting platelet aggregation in humans which method comprises administering an effective, non-toxic amount of a compound of formula (I) or a pharmaceutically acceptable salt or acid addition salt or in vivo hydrolysable acyl derivative thereof to humans in need of such treatment"
Page 31 1st line below "¹H nmr"
   delete "10H, m + 1H replaceable"
   and insert "9H, m + 2H replaceable"
Page 52 under "Mean Food Intake" insert
   "94" for compound of Example 9
   "70" for compound of Example 10

## Claims

1. A compound of formula (I) 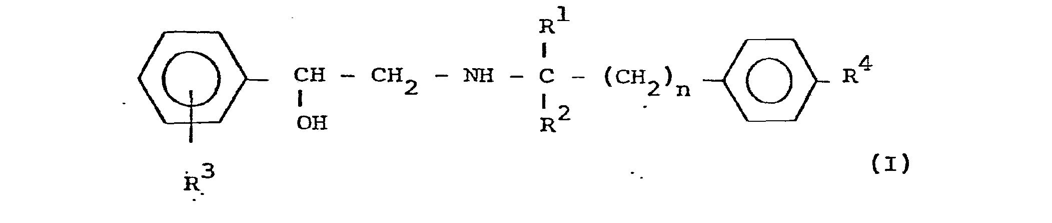
and salts and in vivo hydrolysable acyl derivatives thereof,
wherein
R¹ is hydrogen or methyl;
R² is hydrogen or methyl;
R³ is hydrogen, fluorine, chlorine, bromine, trifluoromethyl or C₁₋₆ straight or branched chain alkyl;
R⁴ is nitro, 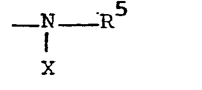
or optionally substituted 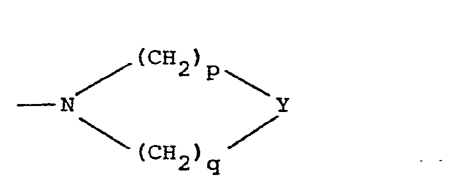
R⁵ is hydrogen or C₁₋₆ straight or branched chain alkyl;
. X is R⁶; 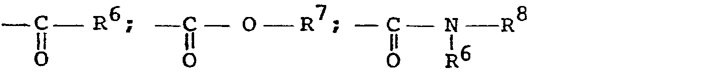 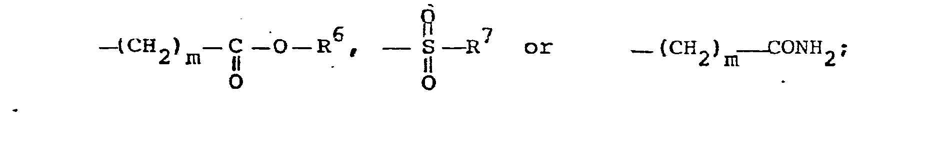
R⁶ is hydrogen or Cₗ-₆ straight or branched chain alkyl;
R⁷ is C₁₋₆ straight or branched chain alkyl;
R⁸ is hydrogen or C₁₋₆ straight or branched chain alkyl;
m is an integer from 1 to 6;
p is an integer from 2 to 5;
q is an integer from 0 to 2;
provided that p + q is from 3 to 5;
Y is 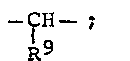 -O-; -S - or 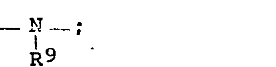
R⁹ is hydrogen, C₁₋₆ straight or branched alkyl or aralkyl consisting of a C₆ or C₁₀ aryl group and a C₁₋₆ straight or branched chain alkylene group;
any substituent on the 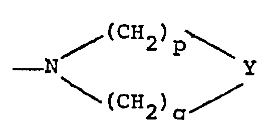 group, when present, being a carboxyl group or a salt, ester or amide thereof.

2. A compound as claimed in claim 1 wherein R³ is hydrogen or trifluoromethyl.

3. A compound as claimed in claim 1 or claim 2 wherein _{R}³ is in the meta position.

4. A compound as claimed in any one of claims 1 to 3 wherein one of R¹ and R³ is methyl and the other is hydrogen.

5. A compound as claimed in any one of claims 1 to 4 wherein R⁴ is nitro or

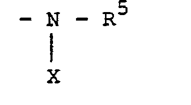

6. A compound as claimed in any one of claims 1 to 4 wherein R⁴ is a cyclic amino group.

7. A process for producing a compound of formula (I), as defined in claim 1, which process comprises either
(a) reducing a compound of formula (II) 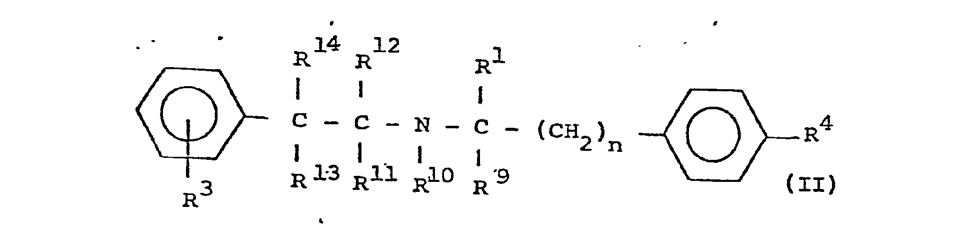
wherein R^{l}, R³, R⁴ and n are as defined in relation to formula (I)
R⁹ is a group R² as defined in relation to formula (I) or together with R¹⁰ forms a bond;
R¹⁰ is hydrogen or together with R⁹ or R¹¹ forms a bond;
R¹¹ is hydrogen or together with R¹² forms an oxo-group or together with R¹⁰ forms a bond;
_{R}¹² is hydrogen or together with R¹¹ forms an oxo-group;
_{R}¹³ is hydroxyl or together with R¹⁴ forms an
oxo-group;
_{R}¹⁴ is hydrogen or together with R¹³ forms an oxo-group, provided that there is no more than one oxo-group and no more than one bond represented by any of R ⁹ to R¹⁴, and optionally thereafter forming a salt of the compound of formula (I) so formed and/or converting the compound of formula (I) so formed into a further compound of formula (I).
or (b) reacting a compound of formula (III) 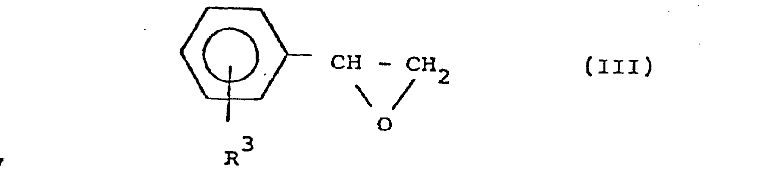 with a compound of formula (IV): 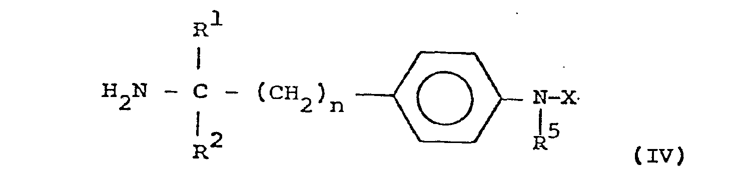 wherein R¹, R R³, R⁵, X and n are as defined in relation to formula (I), and optionally thereafter forming a salt of the compound of formula (I) so formed.

8. A compound of formula (I) as defined in any one of claims 1 to 6 for use in the treatment of the human or animal body.

9. A pharmaceutical composition comprising a compound of formula (I) as defined in any one of claims 1 to 6 in association with a veterinarly acceptable carrier therefor.

10. A process for producing a pharmaceutical composition, as defined in claim 9, which process comprises bringing into association the compound of formula (I) and the carrier.
